# EUROPEAN PATENT APPLICATION

(11) **EP 4 512 901 A1**
(43) Date of publication of application: **26.02.2025**
(21) Application number: 23807771.3
(22) Date of filing: 27.03.2023
(51) Int. Cl.: C12N 15/77, C07K 14/34, C12N 1/20, C12P 13/10

(54) **MICROORGANISM WITH WEAKENED GLUCONATE REPRESSOR PROTEIN ACTIVITY AND METHOD FOR PRODUCING L-ARGININE BY USING SAME**

(30) Priority: 18.05.2022 KR 20220061012
(71) Applicant: CJ CheilJedang Corporation, Seoul 04560 (KR)
(72) Inventor: KIM, Hyo Kyung, Seoul 04560 (KR); CHOI, Sun Hyoung, Seoul 04560 (KR); LEE, Zeewon, Seoul 04560 (KR); SHIM, Jihyun, Seoul 04560 (KR)
(74) Representative: Meissner Bolte Partnerschaft mbB
(86) International application number: PCT/KR2023/004003
(87) International publication number: WO 2023/224248

(57) **Abstract**

The present disclosure relates to a microorganism in which the activity of a gluconate repressor protein is weakened; a method for producing L-arginine using the microorganism of the present disclosure; a composition for producing L-arginine, comprising the microorganism of the present disclosure; and use of the microorganism of the present disclosure for producing L-arginine.

## Description

### [Technical Field]

The present disclosure relates to a microorganism in which the activity of a gluconate repressor protein is weakened; a method for producing L-arginine using the microorganism of the present disclosure; a composition for producing L-arginine, comprising the microorganism of the present disclosure; and use of the microorganism of the present disclosure for producing L-arginine.

### [Background Art]

L-arginine is used for medicines, such as a liver function-improving agent, a brain function-improving agent, and a complex amino acid preparation, and has recently been receiving attention for use in food products, such as a fish cake additive, a health drink additive, and a salt substitute for hypertensive patients. Continuous research is made to use microorganisms in the production of industrially applicable arginine at high concentrations.

Meanwhile, microorganisms of the genus *Corynebacterium*, particularly *Corynebacterium glutamicum*, are Gram-positive microorganisms widely used for the production of L-amino acids. For the production of L-arginine, target substance-specific approaches are primarily used, such as increasing the expression of genes encoding enzymes primarily involved in L-arginine biosynthesis or removing genes unnecessary for L-arginine biosynthesis in a strain of the genus *Corynebacterium* (Korean Patent No. 10-1102263).

However, there is still a growing need for research on methods for efficient production of L-arginine in high yields.

### [Disclosure]

### [Technical Problem]

The present inventors have developed a microorganism of the genus *Corynebacterium* in which the activity of a gluconate repressor protein is weakened, a method for producing L-arginine using the microorganism, a composition for producing L-arginine comprising the microorganism, and use of the microorganism for producing L-arginine, thereby completing the present disclosure.

### [Technical Solution]

An object of the present disclosure is to provide a microorganism of the genus *Corynebacterium* in which the activity of a gluconate repressor protein is weakened.

Another object of the present disclosure is to provide a method for producing L-arginine, comprising culturing the microorganism of the genus *Corynebacterium* of the present disclosure in a medium.

Still another object of the present disclosure is to provide a composition for producing L-arginine, comprising the microorganism of the genus *Corynebacterium* of the present disclosure.

Still another object of the present disclosure is to provide the use of the microorganism of the genus *Corynebacterium* of the present disclosure for producing L-arginine.

### [Advantageous Effects]

The microorganism of the genus *Corynebacterium* of the present disclosure in which the activity of a gluconate repressor protein is weakened is capable of producing L-arginine in high yield compared with a microorganism of the genus *Corynebacterium* in which the activity of a gluconate repressor protein is not weakened.

### [Detailed Description of Preferred Embodiments]

The present disclosure will be described in detail as follows. Meanwhile, each description and embodiment disclosed herein may also be applied to other descriptions and embodiments. That is, all combinations of various elements disclosed herein fall within the scope of the present disclosure. Further, the scope of the present disclosure is not limited by the specific description described below. Furthermore, a number of papers and patent documents are referenced and cited throughout this specification. The disclosures of the cited papers and patent documents are incorporated herein by reference in their entirety to further clarify the level of art and the description of the present disclosure.

An aspect of the present disclosure provides a microorganism of the genus *Corynebacterium* in which the activity of a gluconate repressor protein is weakened.

The microorganism of the genus *Corynebacterium* of the present disclosure may have L-arginine producing ability. Further, the microorganism of the genus *Corynebacterium* of the present disclosure may have increased L-arginine producing ability compared with a microorganism of the genus *Corynebacterium* in which the activity of a gluconate repressor protein is not weakened
As used herein, the term "gluconate repressor (GntR) protein" refers to a regulatory protein involved in gluconate metabolism, sugar uptake, or the like.

Specifically, the gluconate repressor protein is known in the art, and the protein and gene sequences of the gluconate repressor protein may be obtained from a known database, such as GenBank of NCBI, but are not limited thereto. More specifically, the gluconate repressor protein may be a protein consisting of the amino acid sequence of SEQ ID NO: 1. Further, the gluconate repressor protein of the present disclosure may have and/or include the amino acid sequence set forth in SEQ ID NO: 1 or may essentially consist of the amino acid sequence.

Further, the gluconate repressor protein of the present disclosure may include the amino acid sequence set forth in SEQ ID NO: 1, as well as an amino acid sequence having at least 70%, 75%, 80%, 85%, 90%, 95%, 96%, 97%, 98%, 99%, 99.5%, 99.7% or 99.9% homology or identity with the amino acid sequence of SEQ ID NO: 1. Further, it is obvious that a protein having an amino acid sequence with a deletion, modification, substitution, conservative substitution, or addition in a portion thereof may also be included within the scope of the present disclosure, provided that the amino acid sequence has such homology or identity and exhibits a biological activity that is identical or corresponding to the gluconate repressor protein of the present disclosure.

Further, the gluconate repressor protein having the amino acid sequence of SEQ ID NO: 1 may be encoded by a polynucleotide that has or includes the nucleotide sequence of SEQ ID NO: 2 or a nucleotide sequence having 70% or more, 75% or more, 80% or more, 85% or more, 90% or more, 95% or more, 96% or more, 97% or more, or 98% or more, and less than 100% homology or identity with the sequence of SEQ ID NO: 2, or consist of or essentially consist of the base sequence of SEQ ID NO: 2 or a base sequence having 70% or more, 75% or more, 80% or more, 85% or more, 90% or more, 95% or more, 96% or more, 97% or more, or 98% or more, and less than 100% homology or identity with the sequence of SEQ ID NO: 2, but is not limited thereto.

As used herein, the term "L-arginine" refers to an L-amino acid having a chemical formula of C₆H₁₄N₄O₂, which is a conditionally essential amino acid present in all living organisms. L-arginine is known to be produced by microorganisms, mainly by microorganisms of the genus *Corynebacterium*; however, they are also known to be subject to feedback inhibition by intracellular arginine (Vehary Sakanyan, et al., Microbiology, 142:9-108, 1996). Accordingly, it is known that there is a limitation on the high-yield production of L-arginine.

As used herein, the term "microorganism (or strain)" encompasses all of wild-type microorganisms and microorganisms with natural or artificial genetic modification. The term refers to a microorganism in which a particular mechanism is weakened or enhanced due to the insertion of an exogenous gene, the enhancement or inactivation of the activity of an endogenous gene, or the like, which may be a microorganism including a genetic modification for the production of a target polypeptide, protein, or product.

As used herein, the term "microorganism of the genus *Corynebacterium* having L-arginine producing ability" refers to a microbial strain of the genus *Corynebacterium* capable of producing L-arginine within itself, which includes a microorganism in which L-arginine producing ability is imparted to a parent strain with no L-arginine producing ability, or a microorganism having intrinsic L-arginine producing ability. L-arginine producing ability may be imparted or enhanced by species improvement.

As used herein, the term "non-modified microorganism" does not exclude strains including mutations that may naturally occur in microorganisms and may refer to a wild-type strain or native strain as-is, or a strain before its trait is changed due to a genetic mutation caused by natural or artificial factors. For example, the non-modified microorganism may refer to a strain in which the activity of the gluconate repressor protein set forth in this specification is not weakened compared to its intrinsic activity, or a strain prior to the weakening of the activity. The "non-modified microorganism" may be used interchangeably with "strain before modification", "microorganism before modification", "non-mutated strain", "non-modified strain", "non-mutated microorganism" or "reference microorganism".

The non-modified microorganism may be a microorganism including an amino acid sequence consisting of SEQ ID NO: 1 or a polynucleotide consisting of SEQ ID NO: 2.

The microorganism having L-arginine producing ability of the present disclosure may be a genetically modified microorganism or recombinant microorganism, in which the L-arginine producing ability is enhanced due to the weakening of the activity of the gluconate repressor protein compared with the intrinsic activity, but is not limited thereto. Specifically, the recombinant strain with enhanced L-arginine producing ability may be a microorganism in which the L-arginine producing ability is enhanced compared with a natural wild-type microorganism or a non-modified microorganism having the intrinsic activity of a gluconate repressor protein, but is not limited thereto. In an embodiment, the strain with enhanced L-arginine producing ability of the present disclosure may be a microorganism in which the L-arginine producing ability is enhanced compared to a microorganism including the polypeptide of SEQ ID NO: 1 or a polynucleotide encoding the same, but is not limited thereto.

For example, the microorganism having L-arginine producing ability may be a microorganism which inherently contains a gluconate repressor protein.

For example, the microorganism having L-arginine producing ability may be a microorganism inherently containing a polynucleotide sequence capable of encoding the gluconate repressor protein.

In an embodiment, the microorganism with enhanced L-arginine producing ability may have L-arginine producing ability increased by about 1% or more, specifically, about 1% or more, about 2.5% or more, about 5% or more, about 6% or more, about 7% or more, about 8% or more, about 9% or more, about 10% or more, about 10.5% or more, about 11% or more, about 11.5% or more, about 12% or more, about 12.5% or more, about 13% or more, about 13.5% or more, about 14% or more, about 14.5% or more, about 15% or more, about 15.5% or more, about 16% or more, about 16.5% or more, about 17% or more, about 17.5% or more, about 18% or more, about 18.5% or more, or about 19% or more (the upper limit is not particularly limited and may be, for example, about 200% or less, about 150% or less, about 100% or less, about 50% or less, about 45% or less, about 40% or less, about 35% or less, about 30% or less, or about 25% or less) compared with the L-arginine producing ability of the parent strain before modification or non-modified microorganism having intrinsic activity of a gluconate repressor protein, but the L-arginine producing ability is not limited thereto as long as the microorganism has a positive change in the value of the L-arginine producing ability compared with the L-arginine producing ability of the parent strain before modification or non-modified microorganism. In another example, the recombinant strain in which the producing ability is enhanced may have an L-arginine producing ability that is increased by about 1.1 times or more, about 1.12 times or more, about 1.13 times or more, about 1.14 times or more, about 1.15 times or more, about 1.16 times or more, about 1.17 times or more, about 1.18 times or more, or about 1.19 times or more (the upper limit is not particularly limited and may be, for example, about 10 times or less, about 5 times or less, about 3 times or less, about 2 times or less, about 1.5 times or less, or about 1.3 times or less) compared to the parent strain before modification or non-modified microorganism, but the L-arginine producing ability is not limited thereto. As used herein, the term "about" refers to a range encompassing all of ±0.5, ±0.4, ±0.3, ±0.2, ±0.1, and the like, which encompasses all of the values in the range equivalent or similar to those stated after the term "about", but is not limited thereto.

As used herein, the term "a microorganism of the genus *Corynebacterium*" may include all microorganisms of the genus *Corynebacterium.* Specifically, the microorganism of the genus *Corynebacterium* of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris*, or *Corynebacterium flavescens*, more specifically, *Corynebacterium glutamicum.*

While it was already known that a microorganism of the genus *Corynebacterium* is capable of producing L-arginine, the producing ability thereof is significantly low, and the genes involved in the production mechanism or the mechanistic principles have not yet been fully elucidated. Therefore, the microorganism of the genus *Corynebacterium* with L-arginine producing ability of the present disclosure encompasses all of the native wild-type microorganisms, microorganisms of the genus *Corynebacterium* in which the L-arginine producing ability is improved by enhancing or weakening the activity of a gene related to the L-arginine producing mechanism, or microorganisms of the genus *Corynebacterium* in which the L-arginine producing ability is improved by introducing or enhancing the activity of an exogenous gene.

As used herein, the term "weakening" of the activity of a gluconate repressor protein refers to a concept encompassing all of the reduction or absence of the activity compared with the intrinsic activity. The activity of a gluconate repressor protein may be used interchangeably with terms such as "polypeptide activity" and "protein activity". The weakening may be used interchangeably with inactivation, deficiency, down-regulation, decrease, reduction, attenuation, or the like.

The weakening may be inactivation, but is not limited thereto. The inactivation may include all cases in which the gluconate protein activity is less than 100%, 90% or less, 80% or less, 70% or less, 60% or less, 50% or less, 40% or less, 30% or less, 20% or less, 10% or less, 5% or less, or 0% of the intrinsic activity, but is not limited thereto.

The inactivation may refer to a case in which the gluconate repressor protein is not expressed at all, or its activity is absent or reduced even when expressed, compared with the non-modified microorganism.

The weakening may also include: a case in which the activity of the polypeptide is reduced or eliminated compared with the activity of the polypeptide originally possessed by the microorganism due to a mutation in the polynucleotide encoding the polypeptide or the like; a case in which the overall polypeptide activity and/or concentration (expression level) in a cell is lower compared with the same in a native strain due to the inhibition of expression of a gene of a polynucleotide encoding the polypeptide or the inhibition of translation into the polypeptide; a case in which the expression of the polynucleotide is absent; and/or a case in which the protein has no activity despite the expression of the polynucleotide.

The term "intrinsic activity" refers to the activity of a specific polypeptide originally possessed by the parent strain before transformation or wild-type or non-modified microorganism when a trait is changed due to genetic mutation caused by a natural or artificial factor. This term may be used interchangeably with "activity before modification". The "inactivation", "deficiency", "decrease", "down-regulation", "reduction", or "attenuation" of the activity of a polypeptide compared with the intrinsic activity means that the activity of the polypeptide is lowered compared with the activity of a specific polypeptide originally possessed by the parent strain before transformation or non-modified microorganism.

Such weakening of the polypeptide activity may be performed by any method known in the art, but is not limited thereto, and may be attained by applying various methods well known in the art (for example, Nakashima N et al., Bacterial cellular engineering by genome editing and gene silencing. Int J Mol Sci. 2014; 15(2):2773-2793; Sambrook et al., Molecular Cloning 2012, *etc.*).

Specifically, the method of weakening of the activity of a polypeptide of the present disclosure may be:
1) deleting all or a portion of the gene encoding the polypeptide;
2) modifying an expression control region (or expression control sequence) so as to reduce the expression of the gene encoding the polypeptide;
3) modifying an amino acid sequence (for example, deletion/substitution/addition of one or more amino acids in the amino acid sequence) constituting the polypeptide so as to eliminate or weaken the activity of the polypeptide;
4) modifying the gene sequence encoding the polypeptide so as to eliminate or weaken the activity of the protein (for example, deletion/substitution/addition of one or more nucleic acid bases in the nucleic acid base sequence of the polypeptide gene so as to encode the protein modified to eliminate or weaken the activity of the polypeptide);
5) modifying the base sequence encoding the initiation codon or 5'-UTR region of the transcript of the gene encoding the polypeptide;
6) introducing an antisense oligonucleotide (for example, antisense RNA) complementarily binding to the transcript of the gene encoding the polypeptide;
7) adding a sequence complementary to the Shine-Dalgarno sequence of the gene encoding the polypeptide to an upstream region of the Shine-Dalgarno sequence so as to form a secondary structure that makes the attachment of ribosomes impossible;
8) adding a promoter, which is to be reverse-transcribed, to the 3'-end of the open reading frame (ORF) of the gene sequence encoding the polypeptide (reverse transcription engineering, RTE); or
9) a combination of two or more selected from items 1) to 8) above, but is not particularly limited thereto.

For example, these methods may be described as follows:
The deletion of all or a portion of the gene encoding the polypeptide in item 1) above may involve eliminating all of the polynucleotide encoding an endogenous target polypeptide in the chromosome, replacing the polynucleotide with a polynucleotide in which some nucleotides have been deleted, or replacing the same with a marker gene.

The modification of an expression control region (or expression control sequence) in item 2) above may involve inducing a mutation on the expression control region (or expression control sequence) through deletion, insertion, non-conservative or conservative substitution, or a combination thereof, or replacing the same with a sequence having weaker activity. The expression control region includes a promoter, an operator sequence, a sequence for encoding a ribosome binding site, and sequences for controlling the termination of transcription and translation, but is not limited thereto.

The modification of the amino acid sequence or polynucleotide sequence in items 3) and 4) above may involve inducing a mutation into the sequence through deletion, insertion, non-conservative or conservative substitution, or a combination thereof in the amino acid sequence of the polypeptide or the polynucleotide sequence encoding the polypeptide so as to weaken the activity of the polypeptide, or replacing the same with an amino acid sequence or polynucleotide sequence modified to have weaker activity or an amino acid sequence or polynucleotide sequence modified to have no activity, but is not limited thereto. For example, the expression of the gene may be inhibited or weakened by introducing a mutation into the polynucleotide sequence to form a termination codon, but is not limited thereto.

The modification of the base sequence encoding the initiation codon or 5'-UTR region of the transcript of the gene encoding the polypeptide in item 5) above may involve, for example, substituting the base sequence with a base sequence encoding a different initiation codon having a lower polypeptide expression rate compared with the endogenous initiation codon, but is not limited thereto.

The introduction of an antisense oligonucleotide (for example, antisense RNA) complementarily binding to the transcript of the gene encoding the polypeptide in item 6) above may be referred to, for example, the literature [Weintraub, H. et al., Antisense-RNA as a molecular tool for genetic analysis, Reviews - Trends in Genetics, Vol. 1(1) 1986].

The addition of a sequence complementary to the Shine-Dalgarno sequence of the gene encoding the polypeptide to an upstream region of the Shine-Dalgarno sequence so as to form a secondary structure that makes the attachment of ribosomes impossible in item 7) above may involve making mRNA translation impossible or reducing the rate thereof.

The addition of a promoter, which is to be reverse-transcribed, to the 3'-end of the open reading frame (ORF) of the gene sequence encoding the polypeptide (reverse transcription engineering, RTE) in item 8) above may involve making an antisense nucleotide complementary to the transcript of the gene encoding the polypeptide and thereby weakening the activity.

Specifically, the microorganism of the genus *Corynebacterium* having L-arginine producing ability of the present disclosure may include a gluconate repressor variant of an amino acid sequence constituting the gluconate repressor protein, modified so as to eliminate or weaken the activity of the gluconate repressor protein, a polynucleotide encoding the gluconate repressor variant, or a vector comprising the polynucleotide, but is not limited thereto.

More specifically, the gluconate repressor variant may be a gluconate repressor variant, in which one or more amino acids selected from the group consisting of the amino acid corresponding to position 36, the amino acid corresponding to position 59, the amino acid corresponding to position 60, the amino acid corresponding to position 63, the amino acid corresponding to position 79, and the amino acid corresponding to position 92 of the amino acid sequence of SEQ ID NO: 1 are substituted with other amino acids, but is not limited thereto.

As used herein, the term "variant" refers to a polypeptide which has an amino acid sequence different from the amino acid sequence of the variant before modification by conservative substitution and/or modification of one or more amino acids but maintains the functions or properties. Such a variant may generally be identified by modifying one or more amino acids in the amino acid sequence of the polypeptide and evaluating the properties of the modified polypeptide. In other words, the ability of the variant may be increased, unchanged, or decreased as compared to that of the polypeptide before variation. Further, some variants may include a variant in which one or more portions such as an N-terminal leader sequence or a transmembrane domain have been removed. Other variants may include a variant in which a portion of the sequence has been removed from the N-and/or C-terminus of a mature protein. The term "variant" may be used interchangeably with terms such as modification, modified polypeptide, modified protein, mutant, mutein, and divergent and is not limited thereto, provided that the term is used to denote variation.

Further, the variant may include deletions or additions of amino acids that have a minimal effect on the properties and secondary structure of the polypeptide. For example, a signal (or leader) sequence that is co-translationally or post-translationally involved in the protein translocation may be conjugated to the N-terminus of the variant. Further, the variant may be conjugated to other sequences or linkers so as to be identified, purified, or synthesized.

The "other amino acids" are not limited as long as the amino acids are different from the amino acid before substitution. Meanwhile, it is obvious that when the expression "a specific amino acid is substituted" is used in the present disclosure, the amino acid is substituted with an amino acid different from the amino acid before the substitution, even in the absence of a particular description indicating that the amino acid is substituted with a different amino acid.

Amino acids may generally be classified based on the similarity in polarity, charge, solubility, hydrophobicity, hydrophilicity, and/or amphipathic nature of the residues.

As an example of such classification, positively charged (basic) amino acids include arginine, lysine, and histidine; negatively charged (acidic) amino acids include glutamic acid and aspartic acid; amino acids with nonpolar side chains (nonpolar amino acids) include glycine, alanine, valine, leucine, isoleucine, methionine, phenylalanine, tryptophan, and proline; and amino acids with polar or hydrophilic side chains (polar amino acids) include serine, threonine, cysteine, tyrosine, asparagine, and glutamine. As another example, the amino acids may be classified as follows: arginine, lysine, histidine, glutamic acid, and aspartic acid, which are amino acids having electrically charged side chains (electrically charged amino acids); and glycine, alanine, valine, leucine, isoleucine, methionine, phenylalanine, tryptophan, proline, serine, threonine, cysteine, tyrosine, asparagine, and glutamine, which are amino acids having uncharged side chains (uncharged amino acids; also referred to as neutral amino acids). As another example, phenylalanine, tryptophan, and tyrosine may be classified as aromatic amino acids. As another example, valine, leucine, and isoleucine may be classified as branched amino acids. As another example, 20 types of amino acids may be classified into five groups by size, starting from the group of amino acids with relatively small volumes: glycine, alanine, and serine; cysteine, proline, threonine, aspartic acid, and asparagine; valine, histidine, glutamic acid, and glutamine; isoleucine, leucine, methionine, leucine, and arginine; and phenylalanine, tryptophan, and tyrosine. However, the amino acid classification is not limited thereto.

For example, the description "the amino acid corresponding to position 36 of SEQ ID NO: 1 is substituted with another amino acid" may mean that the amino acid is substituted with asparagine, phenylalanine, glycine, alanine, arginine, aspartate, cysteine, glutamate, leucine, glutamine, histidine, proline, serine, tyrosine, lysine, tryptophan, valine, methionine, or threonine, excluding leucine, the amino acid corresponding to position 36 of SEQ ID NO: 1, but is not limited thereto.

It is obvious that when a "polypeptide or protein including an amino acid sequence set forth in a specific SEQ ID NO", "polypeptide or protein consisting of an amino acid sequence set forth in a specific SEQ ID NO", or "polypeptide or protein having an amino acid sequence set forth in a specific SEQ ID NO" is described in the present disclosure, it means that any protein having an amino acid sequence with deletion, modification, substitution, conservative substitution, or addition in a portion thereof may also be used in the present disclosure, provided that the protein has an activity identical or corresponding to the polypeptide consisting of the amino acid sequence of the corresponding SEQ ID NO. For example, this includes addition of a sequence to the N-terminus and/or C-terminus of the amino acid sequence, a naturally-occurring mutation or a silent mutation thereof or conservative substitution thereof that does not alter the functions of the protein.

For example, this includes addition or deletion of a sequence to the N-terminus, C-terminus, and/or within an internal region of the amino acid sequence, naturally-occurring mutation, silent mutation, or conservative substitution that does not alter the functions of the gluconate repressor protein.

As used herein, the term "position N" may include the Nth position and the amino acid position corresponding to the Nth position. Specifically, it may include the amino acid position corresponding to an arbitrary amino acid residue in a mature polypeptide disclosed in a specific amino acid sequence. The specific amino acid sequence may be the amino acid sequence of SEQ ID NO: 1.

As used herein, the term "corresponding to" refers to amino acid residues at positions listed in the polypeptide or amino acid residues that are similar, identical, or homologous to those listed in the polypeptide. Identifying the amino acid at the corresponding position may be determining a specific amino acid in a sequence that refers to a specific sequence. As used herein, the term "corresponding region" generally refers to a similar or corresponding position in a related protein or reference protein.

For example, an arbitrary amino acid sequence may be aligned with SEQ ID NO: 1, and based thereon, each amino acid residue of the arbitrary amino acid sequence may be numbered with reference to the numerical positions of amino acid residues corresponding to the amino acid residues of SEQ ID NO: 1. For example, a sequence alignment algorithm as described in the present disclosure may determine the position of an amino acid or a position at which modification such as substitution, insertion, or deletion occurs through comparison with a query sequence (also referred to as a "reference sequence").

As such alignments, for example, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000), Trends Genet. 16: 276-277), *etc.* may be used, but are not limited thereto, and a sequence alignment program, a pairwise sequence comparison algorithm, *etc.,* which are known in the art, may be appropriately used.

As used herein, the term "conservative substitution" refers to a substitution of an amino acid with another amino acid having similar structural and/or chemical properties. Such amino acid substitution may generally occur based on the similarity in polarity, charge, solubility, hydrophobicity, hydrophilicity, and/or amphipathic nature of the residues. For example, positively charged (basic) amino acids include arginine, lysine, and histidine; negatively charged (acidic) amino acids include glutamic acid and aspartic acid; aromatic amino acids include phenylalanine, tryptophan, and tyrosine; and hydrophobic amino acids include alanine, valine, isoleucine, leucine, methionine, phenylalanine, tyrosine, and tryptophan. Further, amino acids may be classified into amino acids with electrically charged side chains and amino acids with uncharged side chains. The amino acids with electrically charged side chains include aspartic acid, glutamic acid, lysine, arginine, and histidine. The amino acids with uncharged side chains can be further classified into nonpolar amino acids and polar amino acids; nonpolar amino acids include glycine, alanine, valine, leucine, isoleucine, methionine, phenylalanine, tryptophan, and proline, and polar amino acids include serine, threonine, cysteine, tyrosine, asparagine, and glutamine. Typically, conserved substitution has little or no effect on the activity of the produced polypeptide. Typically, conserved substitution may have little or no effect on the activity of the protein or polypeptide.

As used herein, the term "homology" or "identity" refers to the degree of similarity between two given amino acid sequences or base sequences and may be expressed as a percentage. The terms "homology" and "identity" may be often used interchangeably.

The sequence homology or identity of conserved polynucleotides or polypeptides may be determined by the standard alignment algorithm, and default gap penalties established by the program used may be applied together. Substantially, homologous or identical sequences may generally hybridize with each other as a whole or in part under moderate or highly stringent conditions. It is obvious that the hybridization also includes hybridization with a polynucleotide containing usual codons or codons considering codon degeneracy in the polynucleotide.

Whether any two polynucleotide or polypeptide sequences have homology, similarity, or identity may be determined by, for example, a known computer algorithm such as the "FASTA" program using default parameters as in Pearson et al. (1988) [Proc. Natl. Acad. Sci. USA 85]: 2444. Alternatively, they may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including the GCG program package (Devereux, J., et al., Nucleic Acids Research 12: 387 (1984)), BLASTP, BLASTN, FASTA (Atschul, S. F., [et al., J Molec Biol 215]: 403 (1990); Guide to Huge Computers, Martin J. Bishop, [ED.,] Academic Press, San Diego, 1994, and [CARILLO et al.](1988) SIAM J Applied Math 48: 1073). For example, the homology, similarity, or identity may be determined using BLAST of the National Center for Biotechnology Information or ClustalW.

The homology, similarity, or identity between polynucleotides or polypeptides may be determined by comparing sequence information using the GAP computer program as in, for example, Needleman et al., (1970), J Mol Biol. 48:443, as known in Smith and Waterman, Adv. Appl. Math (1981) 2:482. Briefly, the GAP program defines similarity as the number of aligned symbols (i.e., nucleotides or amino acids) which are similar, divided by the total number of symbols in the shorter of the two sequences. The default parameters for the GAP program may include:
(1) a binary comparison matrix (containing a value of 1 for identity and 0 for non-identity) and the weighted comparison matrix of Gribskov et al. (1986) Nucl. Acids Res. 14:6745 as disclosed in Schwartz and Dayhoff, eds., Atlas of Protein Sequence and Structure, National Biomedical Research Foundation, pp. 353-358 (1979) (or the EDNAFULL (EMBOSS version of NCBI NUC4.4) substitution matrix); (2) a penalty of 3.0 for each gap and an additional 0.10 penalty for each symbol in each gap (or a gap open penalty of 10, and a gap extension penalty of 0.5); and (3) no penalty for end gaps.

In an embodiment, the variant may include one or more substitutions selected from the group consisting of the substitution of the amino acid corresponding to position 36 of the amino acid sequence of SEQ ID NO: 1 with asparagine, substitution of the amino acid corresponding to position 59 of the same with alanine, substitution of the amino acid corresponding to position 60 of the same with leucine, substitution of the amino acid corresponding to position 63 of the same with alanine, substitution of the amino acid corresponding to position 79 of the same with alanine, and substitution of the amino acid corresponding to position 92 of the same with leucine, but is not limited thereto.

In one embodiment of the above-described embodiments, the variant provided in the present disclosure may include a substitution of the amino acid corresponding to position 36 from the N-terminus of SEQ ID NO: 1 with a different amino acid.

In one embodiment of the above-described embodiments, the amino acid corresponding to position 36 from the N-terminus of SEQ ID NO: 1 in the variant may be substituted with an amino acid selected from serine, cysteine, tyrosine, asparagine, and glutamine, which are polar amino acids. In one embodiment of the above-described embodiments, the variant may be a variant in which the amino acid corresponding to position 36 from the N-terminus of SEQ ID NO: 1 is substituted with asparagine (N).

In one embodiment of the above-described embodiments, the variant provided in the present disclosure may include a substitution of the amino acid corresponding to position 59 from the N-terminus of SEQ ID NO: 1 with another amino acid.

In one embodiment of the above-described embodiments, the amino acid corresponding to position 59 from the N-terminus of SEQ ID NO: 1 in the variant may be substituted with a nonpolar amino acid. For example, the amino acid may be selected from glycine, alanine, valine, leucine, isoleucine, methionine, phenylalanine, tryptophan, and proline. In one embodiment of the above-described embodiments, the variant may be a variant in which the amino acid corresponding to position 59 from the N-terminus of SEQ ID NO: 1 is substituted with asparagine (N).

In one embodiment of the above-described embodiments, the variant provided in the present disclosure may include a substitution of the amino acid corresponding to position 60 from the N-terminus of SEQ ID NO: 1 with another amino acid.

In one embodiment of the above-described embodiments, the amino acid corresponding to position 60 from the N-terminus of SEQ ID NO: 1 in the variant may be substituted with a nonpolar amino acid. For example, the amino acid may be selected from glycine, alanine, leucine, isoleucine, methionine, phenylalanine, tryptophan, and proline. In one embodiment of the above-described embodiments, the variant may be a variant in which the amino acid corresponding to position 60 from the N-terminus of SEQ ID NO: 1 is substituted with asparagine (N).

In one embodiment of the above-described embodiments, the variant provided in the present disclosure may include a substitution of the amino acid corresponding to position 63 from the N-terminus of SEQ ID NO: 1 with another amino acid.

In one embodiment of the above-described embodiments, the amino acid corresponding to position 63 from the N-terminus of SEQ ID NO: 1 in the variant may be substituted with a nonpolar amino acid. For example, the amino acid may be selected from glycine, alanine, valine, leucine, isoleucine, methionine, phenylalanine, tryptophan, and proline. In one embodiment of the above-described embodiments, the variant may be a variant in which the amino acid corresponding to position 63 from the N-terminus of SEQ ID NO: 1 is substituted with asparagine (N).

In one embodiment of the above-described embodiments, the variant provided in the present disclosure may include a substitution of the amino acid corresponding to position 79 from the N-terminus of SEQ ID NO: 1 with another amino acid.

In one embodiment of the above-described embodiments, the amino acid corresponding to position 79 from the N-terminus of SEQ ID NO: 1 in the variant may be substituted with a nonpolar amino acid. For example, the amino acid may be selected from glycine, alanine, valine, leucine, isoleucine, methionine, phenylalanine, tryptophan, and proline. In one embodiment of the above-described embodiments, the variant may be a variant in which the amino acid corresponding to position 79 from the N-terminus of SEQ ID NO: 1 is substituted with asparagine (N).

In one embodiment of the above-described embodiments, the variant provided in the present disclosure may include a substitution of the amino acid corresponding to position 92 from the N-terminus of SEQ ID NO: 1 with another amino acid.

In one embodiment of the above-described embodiments, the amino acid corresponding to position 92 from the N-terminus of SEQ ID NO: 1 in the variant may be substituted with a nonpolar amino acid. For example, the amino acid may be selected from glycine, alanine, leucine, isoleucine, methionine, phenylalanine, tryptophan, and proline. In one embodiment of the above-described embodiments, the variant may be a variant in which the amino acid corresponding to position 92 from the N-terminus of SEQ ID NO: 1 is substituted with asparagine (N).

Meanwhile, a person skilled in the art would be able to identify, in an arbitrary amino acid sequence, the amino acid corresponding to position 36, amino acid corresponding to position 59, amino acid corresponding to position 60, amino acid corresponding to position 63, amino acid corresponding to position 79, and amino acid corresponding to position 92 in the amino acid sequence of SEQ ID NO: 1 of the present disclosure. Further, when" an amino acid at a specific position of a specific SEQ ID NO" is described, it is obvious that it includes the denotation of the "amino acid at a position corresponding to" a position above in an arbitrary amino acid sequence.

Further, the variant of the present disclosure may include an amino acid sequence having at least 70%, 75%, 80%, 85%, 90%, 95%, 96%, 97%, 98%, 99%, 99.5%, 99.7%, or 99.9% of homology or identity with the amino acid sequence set forth in SEQ ID NO: 1, in which one or more amino acids selected from the group consisting of the amino acids at positions corresponding to position 36, position 59, position 60, position 63, position 79, and position 92 from the N-terminus of SEQ ID NO: 1 are substituted with other amino acids. Further, it is obvious that a variant having an amino acid sequence with deletion, modification, substitution, conservative constitution, or addition of a portion thereof falls within the scope of the present disclosure, provided that the amino acid sequence has such homology or identity and exhibits an effect corresponding to that of the variant of the present disclosure.

For example, the variant of the present disclosure may have or include an amino acid sequence selected from the group consisting of amino acid sequences set forth in SEQ ID NOS: 25 to 31 or may essentially consist of the above amino acid sequences. Alternatively, the variant may include an amino acid sequence having at least 70%, 75%, 80%, 85%, 90%, 95%, 96%, 97%, 98%, 99%, 99.5%, 99.7%, or 99.9% of homology or identity with the amino acid sequence selected from the group consisting of the amino acid sequences set forth in SEQ ID NOS: 25 to 31.

Alternatively, the microorganism of the genus *Corynebacterium* having L-arginine producing ability of the present disclosure may specifically have inactivated gluconate repressor protein activity, but is not limited thereto.

Specifically, a method of deleting all or a portion of the gene encoding the protein may be used to inactivate the activity of the gluconate repressor protein. Specifically, it may be performed by replacing a polynucleotide encoding the endogenous target protein in a chromosome with a polynucleotide, in which a portion of the nucleotide sequence is deleted, or a marker gene, through a vector for chromosomal insertion in a microorganism. As an example of the method for deleting all or a portion of such polynucleotide, a method of deleting the polynucleotide by homologous recombination may be used, but the method is not limited thereto. As another example, a method of deleting the entire or part of the gene may be performed by inducing a mutation using light, such as ultraviolet rays, or chemicals, and selecting a strain in which the target gene is deficient from the resulting variants.

The method of gene deletion includes a method based on genetic recombination technique. For example, it may be performed by inducing homologous recombination through inserting a polynucleotide sequence or vector including a polynucleotide sequence having homology with the target gene into the microorganism. Further, the inserted polynucleotide sequence or vector may comprise a dominant selection marker, but is not limited thereto.

As used herein, the term "polynucleotide" refers to a polymer of nucleotides in which nucleotide monomers are chain-extended lengthwise by covalent bonds, which is a DNA or RNA strand with a certain length, and more specifically, a polynucleotide fragment encoding the protein.

Specifically, the microorganism of the genus *Corynebacterium* having L-arginine producing ability of the present disclosure may include a polynucleotide encoding a modified gluconate repressor variant in which the amino acid sequence constituting the gluconate repressor protein is modified so as to eliminate or weaken the activity of the gluconate repressor protein, but is not limited thereto.

The polynucleotide encoding the gluconate repressor variant may include a sequence without limitation, provided that the sequence is a polynucleotide sequence encoding the gluconate repressor variant of the present disclosure. For example, the polynucleotide encoding the gluconate repressor variant of the present disclosure may be a polynucleotide sequence encoding an amino acid sequence of the variant of the present disclosure, but is not limited thereto.

For example, the polynucleotide encoding the gluconate repressor variant of the present disclosure may be a polynucleotide sequence selected from the group consisting of polynucleotide sequences set forth in SEQ ID NOS: 32 to 38, or a polynucleotide having at least 70%, 80%, 85%, 90%, 95%, 96%, 97%, 98%, or 99% of homology or identity with a polynucleotide sequence selected from the group consisting of polynucleotide sequences set forth in SEQ ID NOS: 32 to 38, but is not limited thereto. A nucleotide sequence having homology or identity may exclude sequences having 100% identity from the scope or may be sequences having less than 100% identity.

The polynucleotide of the present disclosure may have various modifications in the coding region within a scope that does not change the amino acid sequence of the variant of the present disclosure, in consideration of codon degeneracy or the preferred codons in an organism where the variant of the present disclosure is to be expressed. Accordingly, it is obvious that a polynucleotide that may be translated, by codon degeneracy, into a polypeptide consisting of an amino sequence of the variant of the present disclosure or a polypeptide having a homology or identity therewith may also be included.

Further, the polynucleotide of the present disclosure may include any sequence, without limitation, provided that the sequence can hybridize, under stringent conditions, with a probe capable of being prepared from a known gene sequence, for example, a sequence complementary to the entire or a part of the polynucleotide sequence of the present disclosure.

The term "stringent conditions" refers to conditions that enable specific hybridization between polynucleotides. These conditions are specifically described in the literature (see J. Sambrook et al., Molecular Cloning, A Laboratory Manual, 2nd Edition, Cold Spring Harbor Laboratory press, Cold Spring Harbor, New York, 1989; and F. M. Ausubel et al., Current Protocols in Molecular Biology, John Wiley & Sons, Inc., New York, 9.50-9.51, 11.7-11.8). Examples of the stringent conditions may include conditions in which polynucleotides having higher homology or identity, for example, polynucleotides having 70% or more, 75% or more, 80% or more, 85% or more, 90% or more, 95% or more, 96% or more, 97% or more, 98% or more, or 99% or more homology or identity, hybridize with each other, while polynucleotides having lower homology or identity do not hybridize with each other; or conditions of washing of typical Southern hybridization, i.e., conditions of washing once, or two or three times, at a salt concentration and temperature corresponding to 1×SSC, 0.1% SDS at 60°C, specifically 0.1×SSC, 0.1% SDS at 60°C, or more specifically 0.1×SSC, 0.1% SDS at 68°C.

Hybridization requires that two nucleic acids have complementary sequences although mismatches between bases are possible depending on the stringency of the hybridization. The term "complementary" is used to describe the relationship between nucleotide bases that are capable of hybridizing with each another. For example, with respect to DNA, adenine is complementary to thymine, and cytosine is complementary to guanine. Accordingly, the polynucleotide of the present disclosure may also include isolated nucleic acid fragments complementary to the entire sequence as well as substantially similar nucleic acid sequences.

Specifically, polynucleotides having homology or identity with the polynucleotide of the present disclosure may be detected by employing hybridization conditions including a hybridization step at Tm of 55°C and employing the above-described conditions. In addition, the Tₘ value may be 60°C, 63°C, or 65°C, but is not limited thereto, and may be appropriately adjusted by a person skilled in the art according to the purpose.

The appropriate stringency for hybridizing the polynucleotides depends on the lengths and the degree of complementarity of the polynucleotides, and variables are well known in the art (for example, J. Sambrook *et al.*, *supra*).

Alternatively, the microorganism of the genus *Corynebacterium* having L-arginine producing ability of the present disclosure may be a microorganism in which the polynucleotide encoding the gluconate repressor protein is deleted, but is not limited thereto. For example, the microorganism of the genus *Corynebacterium* having L-arginine producing ability of the present disclosure may be a microorganism in which the nucleic acid base sequence of SEQ ID NO: 2 is deleted, but is not limited thereto.

The microorganism of the genus *Corynebacterium* having L-arginine producing ability of the present disclosure may have weakened activity of a gluconate repressor protein compared to the intrinsic activity by including an expression vector for expressing the gluconate repressor variant in the host, but is not limited thereto.

Alternatively, the microorganism of the genus *Corynebacterium* having L-arginine producing ability of the present disclosure may have the gluconate repressor protein activity inactivated by including an expression vector for inactivating the gluconate repressor protein in the host cell, but is not limited thereto.

The vector of the present disclosure may include a DNA construct that include the base sequence of a polynucleotide encoding the target polypeptide, operably linked to an appropriate expression control region (or expression control sequence) so as to express the target polypeptide in an appropriate host. The expression control region may include a promoter capable of initiating transcription, any operator sequence for controlling such transcription, a sequence encoding a suitable mRNA ribosome binding site, and sequences for controlling the termination of transcription and translation. The vector, after transformation into a suitable host cell, may be replicated or function independently of the genome of the host, or may be integrated into the genome itself.

The vector used in the present disclosure is not particularly limited, and any vector known in the art may be used. Commonly used examples of the vector include native or recombinant plasmids, cosmids, viruses, and bacteriophages. For example, pWE15, M13, MBL3, MBL4, IXII, ASHII, APII, t10, t11, Charon4A, and Charon21A may be used as phage vectors or cosmid vectors, and pDZ-based, pBR-based, pUC-based, pBluescriptII-based, pGEM- based, pTZ-based, pCL-based, and pET-based vectors may be used as plasmid vectors. Specifically, pDZ, pDC, pDCM2, pACYC177, pACYC184, pCL, pECCG117, pUC19, pBR322, pMW118, and pCC1BAC vectors may be used.

In an embodiment, a polynucleotide encoding a target polypeptide may be inserted into a chromosome through a vector for chromosomal insertion in a cell. The insertion of the polynucleotide into the chromosome may be performed by using any method known in the art, for example, homologous recombination, but is not limited thereto. The vector may further include a selection marker for confirming the chromosomal insertion. The selection marker is used for selecting vector-transformed cells, that is, confirming the insertion a target nucleic acid molecule, and markers imparting a selectable phenotype, such as drug resistance, auxotrophy, cytotoxic drug resistance, or surface polypeptide expression may be used. In an environment treated with a selective agent, only the cells expressing the selection marker is capable of survival or exhibit a different phenotypic expression, thereby enabling the selection of the transformed cells.

As used herein, the term "transformation" means that a vector including a polynucleotide encoding a target polypeptide is introduced into a host cell or microorganism to enable the polypeptide encoded by the polynucleotide to be expressed in the host cell. The transformed polynucleotide may include any polypeptide as long as it is capable of being expressed in a host cell, regardless of whether it is inserted and located into the chromosome of the host cell or located outside of the chromosome. Further, the polynucleotide includes DNA and/or RNA encoding the target polypeptide. The polynucleotide may be introduced in any form as long as the polynucleotide can be introduced and expressed in a host cell. For example, the polynucleotide may be introduced into the host cell in the form of an expression cassette, which is a gene construct including all factors required for self-expression. The expression cassette may generally include a promoter operably linked to the polynucleotide, a transcription termination signal, a ribosome binding site, and a translation termination signal. The expression cassette may be in the form of an expression vector that is capable of self-replication. Further, the polynucleotide may be introduced into the host cell as-is and operably linked to a sequence required for expression in the host cell, but is not limited thereto.

The term "operably linked" above refers to a functional linkage between a promoter sequence for initiating and mediating the transcription of the polynucleotide encoding the target protein of the present disclosure and the polynucleotide sequence.

In the microorganism of the present disclosure, the modification of all or a portion of the polynucleotide may be induced by (a) homologous recombination using a vector for chromosomal insertion into a microorganism or genome editing using an engineered nuclease (e.g., CRISPR-Cas9) and/or (b) treatment with light, such as UV rays and radioactive rays, and/or chemicals, but is not limited thereto. The method for modifying all or a portion of the gene may include a method using DNA recombinant technology. For example, all or a portion of the gene may be deleted by injecting a nucleotide sequence or vector including a nucleotide sequence having homology with a target gene into the microorganism and thereby inducing homologous recombination. The injected nucleotide sequence or vector may include a dominant selection marker, but is not limited thereto.

Another aspect of the present disclosure provides a method for producing L-arginine, comprising culturing the microorganism of the genus *Corynebacterium* of the present disclosure in a medium.

In the method of the present disclosure, culturing of a microorganism may utilize any culture conditions and culture methods known in the art. Such culturing process may be readily altered and applied by a person skilled in the art according to the selected strain.

As used herein, the term "culturing" means growing the microorganism of the present disclosure under appropriately controlled environmental conditions. The culturing process of the present disclosure may be performed according to suitable medium and culture conditions known in the art. Such culturing process may be readily altered and applied by a person skilled in the art according to the selected strain. Specifically, the culturing may be performed in a batch type, continuous type, and/or fed-batch type mode, but is not limited thereto.

As used herein, the term "medium" means a mixed substance containing nutrients required to culture the microorganism of the present disclosure as a main component, and the medium supplies nutrients, growth factors, *etc.*, including water, which are indispensable for survival and development. Specifically, any medium and other culture conditions may be used for culturing the microorganism of the present disclosure without particular limitation, as long as the medium is commonly used for culturing of a microorganism. The microorganism of the present disclosure may be cultured in a common medium containing proper carbon sources, nitrogen sources, phosphorus sources, inorganic compounds, amino acids and/or vitamins, *etc.*, while controlling the temperature, pH, *etc.* under aerobic conditions.

In the present disclosure, the carbon sources include carbohydrates such as glucose, saccharose, lactose, fructose, sucrose, maltose, *etc.*; sugar alcohols such as mannitol, sorbitol, *etc.*; organic acids such as pyruvic acid, lactic acid, citric acid, *etc.*; and amino acids such as glutamic acid, methionine, lysine, *etc.* Further, natural organic nutrients such as starch hydrolysate, molasses, blackstrap molasses, rice bran, cassava, sugarcane residue, and corn steep liquor may be used. Specifically, carbohydrates such as glucose and sterilized pretreated molasses (*i.e.*, molasses converted to reducing sugar) may be used, and appropriate amounts of other carbon sources may be used in various manners without limitation. These carbon sources may be used alone or in combination of two or more thereof, but are not limited thereto.

As the nitrogen sources, inorganic nitrogen sources such as ammonia, ammonium sulfate, ammonium chloride, ammonium acetate, ammonium phosphate, ammonium carbonate, ammonium nitrate, *etc.*; and organic nitrogen sources such as amino acids such as glutamic acid, methionine, glutamine, *etc.*, peptone, NZ-amine, meat extract, yeast extract, malt extract, corn steep liquor, casein hydrolysate, fish or decomposition products thereof, and skim soybean cake or decomposition products thereof, *etc.* may be used. These nitrogen sources may be used alone or in combination of two or more thereof, but are not limited thereto.

The phosphorus sources may include monopotassium phosphate, dipotassium phosphate, or sodium-containing salts corresponding thereto. As the inorganic compounds, sodium chloride, calcium chloride, iron chloride, magnesium sulfate, iron sulfate, manganese sulfate, calcium carbonate, *etc.* may be used. These components or precursors may be added to the medium in a batch-wise or continuous manner, but the method of addition is not limited thereto.

During the culturing of the microorganism of the present disclosure, the pH of the medium may be adjusted by adding compounds such as ammonium hydroxide, potassium hydroxide, ammonia, phosphoric acid, sulfuric acid, *etc.* to the medium in a suitable manner. Further, during the culturing, foaming may be suppressed by using an anti-foaming agent such as fatty acid polyglycol ester. Further, oxygen or oxygen-containing gas may be injected into the medium in order to maintain the aerobic state of the medium, or no gas may be injected or nitrogen, hydrogen, or carbon dioxide gas may be injected in order to maintain the anaerobic and microaerobic states, but is not limited thereto.

The temperature of the medium may be maintained at 25°C to 40°C, and more specifically, 28°C to 37°C, but is not limited thereto. The culturing may be performed until the desired amount of useful substance is obtained, specifically for 1 to 100 hours, but is not limited thereto.

L-arginine produced by the culturing of the present disclosure may be released into the medium or may remain in cells.

The method for producing L-arginine of the present disclosure may further include preparing the microorganism of the genus *Corynebacterium* of the present disclosure, preparing the medium for culturing the microorganism of the genus *Corynebacterium*, or a combination thereof (in any order), for example, before the culturing step.

The method for producing L-arginine of the present disclosure may further include recovering L-arginine from the medium according to the culturing (a medium in which the culturing was performed) or the microorganism. The method of the present disclosure may further include the recovery step after the culturing step.

The recovery may be collecting desired L-arginine using an appropriate method known in the art according to the method for culturing the microorganism of the present disclosure, for example, a batch, continuous, fed-batch type, or the like. The method may involve, for example, centrifugation, filtration, treatment with a crystallized protein precipitating agent (salting-out), extraction, ultrasonic disruption, ultra-filtration, dialysis, various types of chromatography, such as molecular sieve chromatography (gel filtration), adsorption chromatography, ion exchange chromatography, and affinity chromatography, HPLC, and a combination thereof, and the desired L- arginine may be recovered from the medium or microorganism by using an appropriate method known in the art.

Further, the method for producing L-arginine of the present disclosure may further include a purification step. The purification may be performed using an appropriate method known in the art. In an embodiment, when the method for producing L-arginine of the present disclosure includes both the recovery step and the purification step, the recovery and purification steps may be performed in a continuous or discontinuous manner regardless of the order, or may be performed simultaneously by integrating into one step, but the method is not limited thereto.

Still another aspect of the present disclosure provides a composition for producing L-arginine comprising a microorganism of the genus *Corynebacterium* of the present disclosure.

The composition of the present disclosure may further include any suitable excipients commonly used in compositions for producing L-arginine, and the excipients may include, for example, a preservative, a wetting agent, a dispersing agent, a suspending agent, a buffer, a stabilizer, an isotonic agent, *etc.*, but are not limited thereto.

Still another aspect of the present disclosure provides the use of the microorganism of the genus *Corynebacterium* of the present disclosure for producing L-arginine.

### [Mode for Carrying Out the Invention]

Hereinafter, the present disclosure will be described in detail with reference to examples. However, these examples are merely preferable embodiments for illustrating the present disclosure, and therefore, the examples are not intended to limit the scope of right of the present disclosure. Meanwhile, the technical matters not described herein can be sufficiently understood and easily implemented by a person skilled in the art or related art of the present disclosure.

### Example 1. Preparation of vectors for weakening of gluconate repressor activity

In order to weaken the activity of a gluconate repressor, the gntR1 (136N), gntR1(R59A), gntR1(T60L), gntR1(R63A), gntR1(R79A), and gntR1(A92L) variants, which are represented by SEQ ID NOS: 25 to 31, respectively, were inserted into *Corynebacterium glutamicum* KCCM10741P strain. Accordingly, vectors including the target mutations for weakening the gluconate repressor activity were constructed. Specifically, using the genomic DNA of *Corynebacterium glutamicum* ATCC13869 as a template, PCR using the primer pairs listed in Table 1 (SEQ ID NOS: 3 and 5, SEQ ID NOS: 4 and 6, SEQ ID NOS: 3 and 7, SEQ ID NOS: 4 and 8, SEQ ID NOS: 3and 9, SEQ ID NOS: 4 and 10, SEQ ID NOS: 3 and 11, SEQ ID NOS: 4 and 12, SEQ ID NOS: 3 and 13, SEQ ID NOS: 4 and 14, SEQ ID NOS: 3 and 15, and SEQ ID NOS: 4 and 16) and overlapping PCR using the primer pair SEQ ID NOS: 3 and 4 were performed to obtain homologous recombination fragments with each of the gntR1-136N, R59A, T60L, R63A, R79A, and A92L mutant sequences (hereinafter referred to as "mutation introduced fragment 1" to "mutation introduced fragment 6," respectively). The PCR was performed by repeating 30 cycles of denaturation at 95°C for 30 seconds, annealing at 55°C for 30 seconds, and polymerization at 72°C for one minute.

Subsequently, the pDCM2 vector (Korean Patent Application Publication No. 10-2020-0136813), which cannot be replicated in *Corynebacterium glutamicum*, and the PCR-amplified fragments were treated with the restriction enzymes BamHI and *Xba*I for chromosomal integration. These fragments were then ligated using DNA ligase to construct plasmids with each mutation introduced fragment inserted. Each of these plasmids was transformed into *E. coli* DH5α, which was then plated on LB solid medium containing kanamycin (25 mg/L). Colonies transformed with the plasmids on the LB solid medium were selected, and DNA was extracted using the DNA-spin Plasmid DNA Purification Kit (iNtRON) to produce pDCM2-gntR1 (136N), pDCM2-gntR1 (R59A), pDCM2-gntR1 (T60L), pDCM2-gntR1 (R63A), pDCM2-gntR1(R79A), and pDCM2-gntR1 (A92L) vectors (recombinant plasmids) including the mutation introduced fragments 1 to 6, respectively.

**[Table 1]**

| No. | Name | DNA sequence (5'-3') |
|---|---|---|
| SEQ ID NO: 3 | gntR1-AF | tcggtacccggggatccAAGCAACCGTCGGCGAG |
| SEQ ID NO: 4 | gntR1-BR | tgcaggtcgactctagaTTTCGGGACGCAGTTTC |
| SEQ ID NO: 5 | gntR1 (136N)-AR | AGCAACCTTGCCACTGtTGATATCTTGACCGAGC |
| SEQ ID NO: 6 | gntR1 (136N)-BF | aCAGTGGCAAGGTTGCTGTCGGAGATACCTTCA AG |
| SEQ ID NO: 7 | gntR1 (R59A)-AR | TCGCGTGCCACTGTGgcGGAAATACCAAAACGC |
| SEQ ID NO: 8 | gntR1 (R59A)-BF | gcCACAGTGGCACGCGAAGCAATGCGCGCTTTG |
| SEQ ID NO: 9 | gntR1 (T60L)-AR | GCTTCGCGTGCCACTagGCGGGAAATACCAAAA CG |
| SEQ ID NO: 10 | gntR1 (T60L)-BF | ctAGTGGCACGCGAAGCAATGCGCGCTTTGGAG CA |
| SEQ ID NO: 11 | gntR1 (R63A)-AR | GCGCGCATTGCTTCGgcTGCCACTGTGCGGG |
| SEQ ID NO: 12 | gntR1 (R63A)-BF | gcCGAAGCAATGCGCGCTTTGGAGCAGCTCGGT C |
| SEQ ID NO: 13 | gntR1 (R79A)-AR | GTAATGCCAATGCGAgcTGAAGAAGCGACAAGA CC |
| SEQ ID NO: 14 | gntR1 (R79A)-BF | gcTCGCATTGGCATTACTGTTTTGCCACAGGAAG |
| SEQ ID NO: 15 | gntR1 (A92L)-AR | GACTTATCAAAAACAagCCACTCTTCCTGTGGC |
| SEQ ID NO: 16 | gntR1 (A92L)-BF | ctTGTTTTTGATAAGTCCATCATTCGCTGGCGTC |

### Example 2. Preparation of strains with weakened gluconate repressor activity based on KCCM10741P strain and evaluation of L-arginine producing ability

### Example 2-1. Preparation of strains

In order to weaken the gluconate repressor protein activity in the L-arginine-producing strain *Corynebacterium glutamicum* KCCM10741P (US 8034602 B2), the strain was transformed with each of the recombinant plasmids (pDCM2-gntR1(I36N), pDCM2-gntR1(R59A), pDCM2-gntR1 (T60L), pDCM2-gntR1 (R63A), pDCM2-gntR1 (R79A), and pDCM2-gntR1(A92L)) constructed in Example 1 (van der Rest et al., Appl Microbiol Biotechnol 52:541-545, 1999). Subsequently, secondary recombination was performed on solid plate media containing 4% sucrose, and the transgenic strains that had completed secondary recombination were subjected to PCR using a primer pair(SEQ ID NOS: 3 and 4) to confirm that each mutation had been introduced into the chromosomal gntR1 gene. The PCR was performed by repeating 30 cycles of denaturation at 95°C for 30 seconds, annealing at 55°C for 30 seconds, and polymerization at 72°C for one minute.

Each of the transgenic strains were named KCCM10741P-gntR1(I36N), KCCM10741P-gntR1(R59A), KCCM10741P-gntR1(T60L), KCCM10741P-gntR1(R63A), KCCM10741P-gntR1(R79A), and KCCM10741P-gntR1(A92L), respectively.

### Example 2-2. Evaluation of L-arginine producing ability

In order to compare the L-arginine producing abilities of the parent strain *Corynebacterium glutamicum* KCCM10741P and the transgenic strains constructed in Example 2-1 (strains with weakened gluconate repressor protein activity, KCCM10741P-gntR1(I36N), KCCM10741P-gntR1(R59A), KCCM10741P-gntR1(T60L), KCCM10741P-gntR1(R63A), KCCM10741P-gntR1(R79A), and KCCM10741P-gntR1(A92L)), each strain was cultured by the following method, and the L-arginine concentration in the medium was analyzed.

Each of the parent strain *Corynebacterium glutamicum* KCCM10741P and the transgenic strains constructed in Example 1-2 (strains with weakened gluconate repressor protein activity) was inoculated in a 250-mL corner-baffled flask containing 25 mL of the following seed medium and cultured with shaking at 200 rpm at 30°C for 20 hours. Then, 1 mL of the seed medium was inoculated in a 250-mL corner-baffle flask containing 24 mL of production medium and cultured with shaking at 200 rpm at 30°C for 72 hours. The compositions of the seed medium and production medium used are as follows.

### <Seed medium (pH 7.2)>

Glucose 20 g, Ammonium Sulfate 45 g, Magnesium Sulfate Heptahydrate 2 g, Monopotassium Phosphate 2 g, Ammonium Chloride 10 g, Biotin 0.01 mg, Thiamine-HCl 0.1 mg, Calcium Pantothenate 2 mg, Nicotinamide 3 mg, Ferrous Sulfate 10 mg, Manganese Sulfate 10 mg, Zinc Sulfate 0.02 mg, Copper Sulfate 0.5 mg (based on 1 liter of distilled water).

### <Production medium (pH 7.2)>

Glucose 60 g, Ammonium Sulfate 45 g, Magnesium Sulfate Heptahydrate 2 g, Monopotassium Phosphate 2 g, Ammonium Chloride 10 g, Biotin 0.01 mg, Thiamine-HCl 0.1 mg, Calcium Pantothenate 2 mg, Nicotinamide 3 mg, Ferrous Sulfate 10 mg, Manganese Sulfate 10 mg, Zinc Sulfate 0.02 mg, Copper Sulfate 0.5 mg, Calcium Carbonate 30g (based on 1 liter of distilled water).

After the completion of the culture, the L-arginine producing ability (concentration) was analyzed using HPLC (Waters 2478), and the obtained L-arginine concentrations are shown in Table 2 below.

**[Table 2]**

| | Name of Strain | L-arginine (g/L) | Batch 2 | Batch 3 | Mean |
|---|---|---|---|---|---|
| Control Group | KCCM10741P | 2.9 | 3.0 | 3.1 | 3.0 |
| Experimental group | KCCM10741P-gntR1 (136N) | 3.4 | 3.6 | 3.5 | 3.5 |
| | KCCM10741P-gntR1 (R59A) | 3.6 | 3.3 | 3.5 | 3.5 |
| | KCCM10741P-gntR1 (T60L) | 3.4 | 3.5 | 3.5 | 3.5 |
| | KCCM10741P-gntR1 (R63A) | 3.6 | 3.5 | 3.0 | 3.4 |
| | KCCM10741P-gntR1 (R79A) | 3.4 | 3.7 | 3.5 | 3.5 |
| | KCCM10741P-gntR1 (A92L) | 3.6 | 3.5 | 3.3 | 3.5 |

As a result, it was confirmed that the L-arginine producing ability increased by an average of about 17% in all of the transgenic strains (strains with weakened gluconate repressor protein activity; KCCM10741P-gntR1(I36N), KCCM10741P-gntR1(R59A), KCCM10741P-gntR1(T60L), KCCM10741P-gntR1(R63A), KCCM10741P-gntR1(R79A), and KCCM10741P-gntR1 (A92L)) compared to that of the parent strain.

### Example 3. Preparation of strains with weakened gluconate repressor protein activity based on CJ1R strain and evaluation of L-arginine producing ability

### Example 3-1. Preparation of CJ1R strain

In order to determine whether the effect of increasing L-arginine producing ability was also observed in other *Corynebacterium glutamicum* strains having L-arginine producing ability, a *Corynebacterium glutamicum strain* CJ1R having L-arginine producing ability was additionally constructed by introducing one mutation (ΔargR) into the wild-type strain (ATCC13869). Specifically, using the genomic DNA of *Corynebacterium glutamicum* ATCC13869 as a template, PCR using the primer pairs in Table 3 (SEQ ID NOS: 17 and 18, and SEQ ID NOS: 19 and 20) and overlapping PCR using the primer pair of SEQ ID NOS: 17 and 20 were performed to obtain homologous recombination fragments having an argR deficient mutant sequence. The PCR was performed by repeating 30 cycles of denaturation at 95°C for 30 seconds, annealing at 55°C for 30 seconds, and polymerization at 72°C for one minute.

The constructed vector (recombinant plasmid) was named pDCM2-ΔargR.

**[Table 3]**

| No. | Name | DNA sequence (5'-3') |
|---|---|---|
| SEQ ID NO: 17 | argR-AF | tcggtacccggggatccAGCAGGCCTTAAGGGTAAG |
| SEQ ID NO: 18 | argR-AR | AGGGGCGCTGTCTTACCTCGGCTGGTGGGCCAGC |
| SEQ ID NO: 19 | argR-BF | GGTAAGACAGCGCCCCTAGTTCAAGGCTTGTTAATC |
| SEQ ID NO: 20 | argR-BR | tgcaggtcgactctagaACCGTTGAACTGCTTGCCAG |

Subsequently, the constructed pDCM2-ΔargR vector was transformed into the wild-type *Corynebacterium glutamicum* strain (ATCC13869) (van der Rest et al., Appl Microbiol Biotechnol 52:541-545, 1999), and then, secondary recombination was performed on a solid plate medium containing 4% sucrose. The strain with argR deleted from the chromosome was confirmed by subjecting the transgenic strain that had completed the secondary recombination to PCR using a primer pair (SEQ ID NOS: 19 and 22). The PCR was performed by repeating 30 cycles of denaturation at 95°C for 30 seconds, annealing at 55°C for 30 seconds, and polymerization at 72°C for one minute. The transgenic strain was named CJ1R.

### Example 3-2. Preparation of strains with weakened gluconate repressor activity based on CJ1R strain

In order to weaken the gluconate repressor protein activity of the CJ1R strain, mutated strains were constructed by introducing each of the vectors produced in Example 1 in the same manner as in Example 2-1, and the mutant strains were named CJ1R-gntR1(I36N), CJ1R-gntR1(R59A), CJ1R-gntR1(T60L), CJ1R-gntR1(R63A), CJ1R-gntR1(R79A), and CJ1R-gntR1(A92L), respectively.

### Example 3-3. Evaluation of L-arginine producing ability

In order to compare the L-arginine producing abilities of the L-arginine producing strain *Corynebacterium glutamicum* CJ1R and the mutated strains constructed in Example 3-2 (strains with weakened gluconate repressor protein activity; CJ1R-gntR1(I36N), CJ1R-gntR1(R59A), CJ1R-gntR1(T60L), CJ1R-gntR1(R63A), CJ1R-gntR1(R79A), and CJ1R-gntR1 (A92L)), each strain was cultured in the same manner as in Example 2-2, and the L-arginine concentration in the medium was determined.

After the completion of the culture, the L-arginine producing ability (concentration) was analyzed using HPLC (Waters 2478), and the obtained L-arginine concentrations are shown in Table 4 below.

**[Table 4]**

| | Name of Strain | L-arginine (g/L) | | | |
|---|---|---|---|---|---|
| | | Batch 1 | Batch 2 | Batch 3 | Mean |
| Control group | CJ1R | 2.0 | 2.2 | 2.1 | 2.1 |
| Experimental group | CJ1R-gntR1 (136N) | 2.5 | 2.7 | 2.5 | 2.6 |
| | CJ1R-gntR1 (R59A) | 2.3 | 2.6 | 2.8 | 2.6 |
| | CJ1R-gntR1 (T60L) | 2.6 | 2.5 | 2.4 | 2.5 |
| | CJ1R-gntR1 (R63A) | 2.4 | 2.4 | 2.6 | 2.5 |
| | CJ1R-gntR1 (R79A) | 2.5 | 2.6 | 2.7 | 2.6 |
| | CJ1R-gntR1 (A92L) | 2.5 | 2.5 | 2.3 | 2.4 |

As a result, it was confirmed that the L-arginine producing ability increased by an average of about 19% in all of the mutated strains (strains with weakened gluconate repressor protein activity; CJ1R-gntR1(I36N), CJ1R-gntR1(R59A), CJ1R-gntR1(T60L), CJ1R-gntR1(R63A), CJ1R-gntR1(R79A), and CJ1R-gntR1(A92L)) compared to the parent strain.

### Example 4. Preparation of strains with weakened gluconate repressor activity and evaluation of L-arginine producing ability

### Example 4-1. Preparation of strains

In order to determine the effect of weakening the gluconate repressor activity on L-arginine production, strains were constructed by deleting the gntR1 gene in the *Corynebacterium glutamicum* strains KCCM10741P and CJ1R, both of which possess L-arginine producing ability.

Specifically, a recombinant vector for deleting the gntR1 gene was constructed in the same manner as in Example 3-1, and the primers used for the construction of the vector are shown in Table 5. Specifically, using the genomic DNA of *Corynebacterium glutamicum* ATCC13869 as a template, PCR using the primer pairs in Table 5 (SEQ ID NOS: 21 and 22, and SEQ ID NOS: 23 and 24) and overlapping PCR using the primer pair of SEQ ID NOS: 21 and 24 were performed to obtain homologous recombination fragments having an argR deficient mutant sequence. The PCR was performed by repeating 30 cycles of denaturation at 95°C for 30 seconds, annealing at 55°C for 30 seconds, and polymerization at 72°C for one minute.

The constructed vector (recombinant plasmid) was named pDCM2-ΔgntR1.

**[Table 5]**

| No. | Name | DNA sequence (5'-3') |
|---|---|---|
| SEQ ID NO: 21 | gntR1 del-AF | tcggtacccggggatccTTGACCGTGACTACACC |
| SEQ ID NO: 22 | gntR1 del-AR | GTATCACGGGTGCCTCTTTAATGGGCCGGTGTAC |
| SEQ ID NO: 23 | gntR1 del-BF | AGAGGCACCCGTGATACGCGCACTGCGTTTGCC A |
| SEQ ID NO: 24 | gntR1 del-BR | tgcaggtcgactctagaGGCTTCGTCAATTACCG |

Subsequently, the constructed pDCM2-ΔgntR1 vector was transformed into the *Corynebacterium glutamicum* strains KCCM10741P and CJ1R, both of which possess L-arginine producing ability (van der Rest et al., Appl Microbiol Biotechnol 52:541-545, 1999), and then, secondary recombination was performed on a solid plate medium containing 4% sucrose. The strain with gntR1 deletion on the chromosome was confirmed by subjecting the transgenic strain that had completed the secondary recombination to PCR using a primer pair (SEQ ID NOS: 21 and 24). The PCR was performed by repeating 30 cycles of denaturation at 95°C for 30 seconds, annealing at 55°C for 30 seconds, and polymerization at 72°C for one minute. The transgenic strains were named KCCM10741P-ΔgntR1 and CJ1R-ΔgntR1, respectively.

### Example 4-2. Evaluation of L-arginine producing ability

In order to compare the L-arginine producing abilities of the L-arginine producing *Corynebacterium glutamicum* strains, KCCM10741P and CJ1R, and the gntR1-deficient strains constructed in Example 4-1 (KCCM10741P-ΔgntR1 and CJ1R-ΔgntR1), each strain was cultured in the same manner as in Example 2-2, and the L-arginine concentration in the medium was determined.

After the completion of the culture, the L-arginine producing ability (concentration) was analyzed using HPLC (Waters 2478), and the obtained L-arginine concentrations are shown in Table 6 below.

**[Table 6]**

| | Name of Strain | L-arginine (g/L) | | | |
|---|---|---|---|---|---|
| | | Batch 1 | Batch 2 | Batch 3 | Mean |
| Control group | KCCM10741P | 3.0 | 3.0 | 3.1 | 3.0 |
| Experimental group | KCCM10741P-ΔgntR1 | 3.3 | 3.4 | 3.5 | 3.4 |
| Control group | CJ1R | 2.2 | 2.2 | 2.0 | 2.1 |
| Experimental group | CJ1R-ΔgntR1 | 2.6 | 2.4 | 2.5 | 2.5 |

As a result, it was confirmed that the L-arginine producing ability increased by an average of 13% to 19% in all of the gntR1-deficient strains (KCCM10741P-ΔgntR1 and CJ1R-ΔgntR1) compared to the parent strains.

As set forth above, a person skilled in the art will be able to understand that the present disclosure may be embodied in other specific forms without departing from the technical spirit or essential characteristics thereof. In relation thereto, the embodiments described above should be construed as being exemplified and not limiting the present disclosure. The scope of the present disclosure should be understood to include all changes or modifications derived from the definitions and scopes of the claims and their equivalents, rather than from the detailed description.

## Claims

1. A microorganism of the genus *Corynebacterium*, wherein the activity of a gluconate repressor protein is weakened.

2. The microorganism of the genus *Corynebacterium* of claim 1, wherein the microorganism has L-arginine producing ability.

3. The microorganism of the genus *Corynebacterium* of claim 1, wherein the gluconate repressor protein consists of an amino acid sequence of SEQ ID NO: 1.

4. The microorganism of the genus *Corynebacterium* of claim 1, wherein the microorganism of the genus *Corynebacterium* is *Corynebacterium glutamicum.*

5. The microorganism of any one of claims 1 to 4, wherein the microorganism has increased L-arginine producing ability.

6. The method for producing L-arginine, comprising culturing, in a medium, a microorganism of the genus *Corynebacterium* in which the gluconate repressor protein activity is weakened.
